# EUROPÄISCHE PATENTSCHRIFT

(11) **EP 3 110 376 B1**
(45) Veröffentlichungstag und Bekanntmachung des Hinweises auf die Patenterteilung: **04.04.2018**
(21) Anmeldenummer: 15711057.8
(22) Anmeldetag: 23.02.2015
(51) Int. Cl.: A61F 9/007

(54) **GLAUKOM-DRAINAGE-IMPLANTAT**
GLAUCOMA DRAINAGE IMPLANT
IMPLANT DE DRAINAGE DE GLAUCOME

(30) Priorität: 25.02.2014 DE 102014102457
(43) Veröffentlichungstag der Anmeldung: 04.01.2017
(73) Patentinhaber: Universität Rostock, 18055 Rostock (DE)
(72) Erfinder: SIEWERT, Stefan, 18055 Rostock (DE); LUDERER, Frank, 18106 Rostock (DE); SCHMIDT, Wolfram, 18109 Rostock (DE); LÖBLER, Marian, 18055 Rostock (DE); GUTHOFF, Rudolf, 18119 Rostock (DE); STERNBERG, Katrin, 78166 Donaueschingen (DE); SCHMITZ, Klaus-Peter, 18119 Rostock (DE)
(74) Vertreter: Garrels, Sabine
(86) Internationale Anmeldenummer: PCT/EP2015/053706
(87) Internationale Veröffentlichungsnummer: WO 2015/128281

(56) Entgegenhaltungen:
- WO-A2-2012/071476
- DE-A1-102007 004 906
- US-A1- 2010 010 416
- US-A1- 2012 035 525

## Beschreibung

Die vorliegende Erfindung bezieht sich auf ein Augenimplantat zur Glaukomtherapie.

Als Glaukom wird eine optische Neuropathie bezeichnet, die mit fortschreitendem Krankheitsverlauf zu charakteristischen Gesichtsfeldeinschränkungen bis hin zur vollständigen Erblindung des betroffenen Auges führen kann. Das Glaukom stellt weltweit die zweithäufigste Ursache für irreversible Erblindungen dar. Als Hauptrisikofaktor für die Entwicklung eines Glaukoms gilt ein dauerhaft über 21 mmHg erhöhter intraokularer Druck (IOD). Die Regelung des intraokularen Drucks erfolgt im gesunden Auge durch die Zirkulation von Kammerwasser, das im Ziliarepithel mit einer Rate von ca. 2 µl/min gebildet wird, von der Hinterkammer über die Iris in die Vorderkammer des Auges strömt und von dort über das Trabekelmaschenwerk in den Schlemm-Kanal abgeleitet wird. Unter physiologischen Bedingungen beträgt der intraokulare Druck ca. 15 mmHg. Der intraokulare Druck resultiert aus der Bilanz zwischen der Kammerwasserproduktion und dem Kammerwasserabtransport. Der Kammerwasserabtransport hängt dabei maßgeblich vom Abflusswiderstand im Trabekelmaschenwerk ab. Im Falle der häufigsten Glaukomform, dem primär chronischen Offenwinkelglaukom, kommt es aufgrund einer Erhöhung des Abflusswiderstands im Trabekelmaschenwerk zu einer Erhöhung des intraokularen Drucks.

Therapieverfahren des Glaukoms zielen generell auf eine Verminderung des intraokularen Drucks auf ein physiologisches Niveau ab. Dies geschieht in erster Linie medikamentös. Für den Fall, dass medikamentös keine ausreichende IOD-Senkung erreicht wird oder Nebenwirkungen eine Fortführung der Therapie behindern, stehen alternativ Laserverfahren wie die Argon-Laser-Trabekuloplastik zur Verminderung des Abflusswiderstands im Trabekelmaschenwerk oder mikrochirurgische Verfahren wie die Trabekulektomie zur Präparation eines zusätzlichen Abflusswegs für das Kammerwasser unter die Bindehaut zur Verfügung. Die Trabekulektomie stellt derzeit noch den Goldstandard bei der Therapie refraktärer Glaukome dar. Als entscheidender Nachteil des Verfahrens ist die starke Abhängigkeit des Therapieerfolgs vom jeweiligen Operateur zu nennen. In diesem Zusammenhang stellen sogenannte Glaukom-Drainage-Implantate (GDI) eine vielversprechende Alternative dar, da der Abflusswiderstand bereits bei der Implantat-Entwicklung festgelegt werden kann.

Auf dem Markt existiert eine Vielzahl kommerzieller Glaukom-Drainage-Implantate. Darunter befinden sich auch Implantate, die minimalinvasiv z.B. im Rahmen einer Kataraktoperation applizierbar sind. Als eine Hauptkomplikation beim Einsatz von Glaukom-Drainage-Implantaten ist die Hypotonie des Auges aufgrund einer übermäßigen Kammerwasserdrainage bekannt. Technische Lösungen dieses Problems werden umfassend in der Patentliteratur beschrieben. Zur Vermeidung der Hypotonie werden hier z.B. komplexe, durch den Arzt extern justierbare, mechatronische Ventilmechanismen vorgeschlagen. Eine technische Umsetzung unter Berücksichtigung wirtschaftlicher Aspekte erscheint kaum möglich. Andere vorgeschlagene Ventilmechanismen zur Vermeidung der Hypotonie arbeiten nach dem Prinzip des Rückschlag-, Membran-, Schlitz- oder Zungenventils und sind damit relativ einfach herstellbar. Neben diesen Ventilmechanismen wurden auch bereits Verschlussmechanismen vorgestellt, die in der frühen postoperativen Phase einer Hypotonie entgegenwirken und zu einem späteren Zeitpunkt z.B. mittels einer Laserbehandlung entfernt werden.

Beispielsweise betrifft DE 102007004906 A1 einen Glaukomstent, welcher einerseits den Augeninnendruck reguliert und andererseits verhindert, dass sich der Flusswiderstand mit der Zeit erhöht. Ein Röhrchen mit einer Wandfläche, welche aus einem flüssigkeitsdichten Material ausgebildet ist, wird vom Augenkammerwasser durchströmt, wobei im Bereich der Wandfläche mindestens ein druckgesteuertes Ventil angeordnet ist.

Allen Ventilmechanismen gemein ist jedoch die Gefahr des Funktionsverlustes mit fortschreitender Implantationszeit, z.B. durch Verschleiß oder Verkleben. Aus der klinischen Praxis ist bekannt, dass die Gefahr einer Hypotonie in der frühen postoperativen Phase am größten ist.

In EP 532654 B1 (DE69120949) wird ein Implantat zur Anwendung bei der Behandlung von Glaukom offenbart, welches eine flexible Platte zum Vernähen mit einem skleralen Bereich des Auges und ein von dieser Platte abgehendes Rohr zur Verbindung mit der vorderen Kammer aufweist. Um das Kammerwassers kontrolliert abfließen zu lassen, wird in einem Ausführungsbeispiel das Drainagerohr mit einer Naht bzw. Nähten solange verschlossen bis sich ein Gewebebläschen über der Trägerplatte gebildet hat. Danach wird die Naht entfern oder es wird eine sich auflösende Naht verwendet. In einer alternativen Ausführungsform ist das Drainagerohr als Doppelrohr mit einem ersten Lumen und einem zweiten, wesentlich kleineren Lumen ausgebildet. In dem ersten Lumen ist ein sich langsam auflösender Stopfen eingesetzt und das zweite Lumen ist mit einem Strömungsbegrenzer versehen, um den Kammerwasserfluss allmählich zu ermöglichen. Ebenso wird als Alternative in einem zweiten Auslassrohr ein Mikrofilter oder ein Strömungsbegrenzer bzw. Ventil eingesetzt, um einen verhältnismäßig kleinen Anfangsstrom zwischen der vorderen Kammer und dem Bläschen zu ermöglichen.

In US 2010137981 A1 wird eine Vorrichtung zur Behandlung eines Glaukoms offenbart, welches aus einem länglichen, hohlen Element mit einem ersten Teil mit einer geflochtenen Struktur und einen zweiten Abschnitt zumindest teilweise aus einer nicht-geflochtenen Struktur gebildet ist. Die geflochtene Struktur kann beim Einsetzen verformt werden, was förderlich für die Retention im Auge ist und den Fluidstrom von der vorderen Kammer verbessert.

Ein Drainageimplantat zur Glaukomtherapie, welches einen Flusswiderstand in Form einer porösen, semipermeablen Membran aufweist, ist in WO 98/30181 A1 beschrieben. Durch den Flusswiderstand soll bereits direkt nach der Implantation der Vorrichtung eine geringe Drainagewirkung erzielt werden. Nach der Bildung einer fibrösen Gewebekapsel um das Implantat, die den Hauptwiderstand der Drainage darstellt kann der Flusswiderstand mittels eines ophthalmischen Lasers teilweise oder vollständig entfernt werden.

In US 2012/0089073 A1 wird ein Barriere beschrieben, die mindestens eines von potentiell mehreren Lumen eines Drainageimplantates zur Glaukomtherapie initial verschließt und so einer Hypotonie entgegenwirkt. Mit zunehmender Implantationszeit soll eine Freigabe des initial verschlossenen Lumens bzw. der initial verschlossenen Lumen z.B. durch die Zersetzung der Barriere erfolgen.

In US 2010/0010416 A1 wird ein Drainageimplantat zur Glaukomtherapie vorgestellt, das eine Kammerwasserdrainage aus der Vorderkammer des Auges in den Suprachoroidalraum ermöglicht. Verschiedene Fixierungsstrukturen wie z.B. Vorsprünge oder Flügel, die eine mechanische Fixierung des Implantates im Auge ermöglichen sollen, werden beschrieben.

Ein in WO 2005/117780 A2 vorgestelltes Augenimplantat dient zur Kammerwasserdrainage sowie als Schnittstelle zur Zufuhr von Medikamenten in die Vorder- oder Hinterkammer des Auges. Das Implantat soll eine Injektion oder Infusion z.B. von Anti-Glaukom-Medikamenten ermöglichen. Zur Fixierung des Implantates im okulären Gewebe wird darüber hinaus eine Beschichtung beschrieben, die eine Anhaftung von Zellen begünstigen soll.

Die Veröffentlichungen WO 2012/071476 A2 und US 2012/035525 A1 befassen sich ebenfalls mit Glaukom-Drainage-Implantaten, bei welchen im Einstrombereich des Implantats eine flusslimitierende Membran angeordnet ist.

Eine Methode zur Herstellung länglicher, hohler Strukturen wird in WO 2004/071736 A2 beschrieben. Die Methode basiert auf der durch Zentrifugalkräfte hervorgerufenen Phasenseparation von Lösungen oder Emulsionen innerhalb einer rotierenden Form.

In WO 99/32536 A1 wird Poly-4-hydroxybuttersäure (P(4HB)) als biokompatibler Polymerwerkstoff mit steuerbaren Degradationseigenschaften beschrieben.

### Darstellung der Erfindung

Die vorliegende Erfindung hat die Aufgabe, zur Senkung des intraokularen Drucks beim Glaukom eine Kammerwasserdrainage aus der Vorderkammer des Auges z.B. in den Subconjunctival oder Suprachoroidalraum zu ermöglichen. Die Vorrichtung soll die Nachteile bekannter Ventilmechanismen umgehen und wirtschaftlich hergestellt werden.

Erfindungsgemäß wird die Aufgabe durch die wesentlichen Merkmale in den Ansprüchen gelöst. Dazu besteht das Glaukom-Drainage-Implantat aus einem länglichen, hohlen Grundkörper, bei dem im Einstrombereich eine flusslimitierende Membran angeordnet ist und an einem vorderen, geschlossenen Ende des Grundkörpers um seinen Umfang verteilt Bohrungen angeordnet sind, welche durch die darüber liegende Membran abgedeckt werden.

Für eine wesentliche Ausführungsform sind lokale Nuten in der Wandfläche des Grundkörpers zur Aufnahme der Membran, eines Fixierungselements und eines Local-Drug-Delivery-Systems entsprechend eingelassen.

Die Membran besteht für eine weitere Ausführungsform aus biodegradierbarem, semipermeablem Material. Dieses Material kann ein Polymerwerkstoff, beispielsweise Poly(4-hydroxybuttersäure), sein.

Für eine weitere Ausführungsform weist die Membran mit zunehmender Implantationszeit einen abnehmenden Strömungswiderstand auf.

Die Membran aus einem biostabilen, semipermeablen Material ist eine weitere Ausführungsform.

Für ein erfindungsgemäßes Glaukom-Drainage-Implantat in einem weiteren Ausführungsbeispiel ist auf ca. einem Drittel des rohrförmigen Grundkörpers von einem vorderen Ende aus gesehen ein Fixierungselement angeordnet, welches zur Fixierung des Implantats im Gewebe dient.

Zur Vermeidung einer Bildung einer fibrösen Gewebekapsel ist an einem hinteren Ende des Grundkörpers ein Local-Drug-Delivery-System angeordnet.

Zur Herstellung der Membran, des Fixierungselements und des Local-Drug-Delivery-Systems kommt ein Fertigungsverfahren in Form von Electrospinning zur Anwendung.

Der Einstrombereich des Implantats wird in die Vorderkammer des Auges und der Ausstrombereich des Implantats in den Suprachoroidal- oder Subconjunctivalraum implantiert.

Das erfindungsgemäße Glaukom-Drainage-Implantat mit einem temporär erhöhten Strömungswiderstand stellt eine vielversprechende Alternative zu mechanischen Ventilmechanismen dar.

### Ausführung der Erfindung

Die Erfindung wird anhand von Ausführungsbeispielen näher erläutert. Hierzu zeigen
Figur 1 eine schematische Darstellung des Auges mit zwei möglichen Drainagen aus der Vorderkammer des Auges mit
Figur 1a einer Drainage in den Subconjunctivalraum oder
Figur 1b einer Drainage in den Suprachoroidalraum,
Figur 2 einer ersten Ausführungsform eines erfindungsgemäßen Glaukom-Drainage-Implantats,
Figur 3 eine Schnittdarstellung aus Figur 2,
Figur 4 einer Detailansicht A aus Figur 3,
Figur 5 einer Detailansicht B aus Figur 3,
Figur 6 einer Detailansicht C aus Figur 3,
Figur 7 einer zweiten Ausführungsform des erfindungsgemäßen Glaukom-Drainage-Implantats,
Figur 8 eine Schnittdarstellung aus Figur 7,
Figur 9 eine Detailansicht D aus Figur 8,
Figur 10 ein Schnitt E-E aus Figur 7,
Figur 11 ein Injektor mit enthaltenem Glaukom-Drainage-Implantat,
Figur 12 eine Detailansicht F aus Figur 11 und
Figur 13 eine Darstellung einer möglichen minimalinvasiven Implantation mit den schematisierten Phasen a und b.

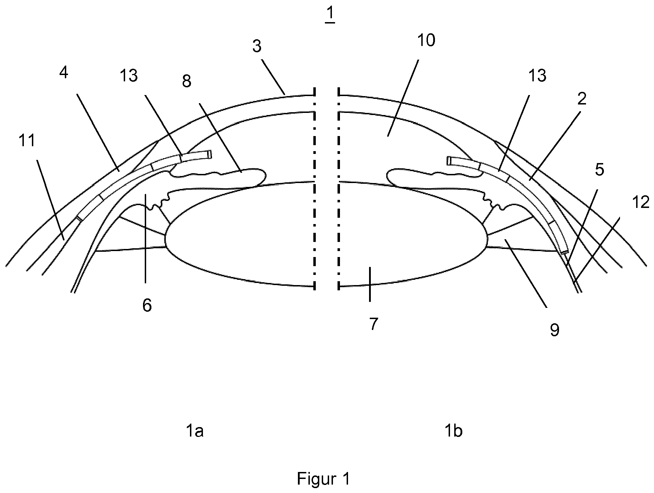

In Figur 1 wird schematisch das Auge dargestellt. Der hier beschriebene Aufbau des Auges 1 beschränkt sich auf die wesentlichen für die Erfindung relevanten Teile (welche für die Figuren 1a und 1b gleichlautend sind) mit der Sclera 2, der Hornhaut 3 (Cornea), der Bindehaut 4 (Conjunktiva), der Aderhaut 5 (Choroidea), dem Ziliarkörper 6, der Linse 7, der Iris 8 und den Zonulafasern 9. Weiterhin werden schematisch zwei möglichen Anordnungen zur Senkung des intraokularen Drucks beim Glaukom mit einem Glaukom-Drainage-Implantat 13 (kurz auch Implantat) aus der Vorderkammer 10 des Auges in den Subconjunctivalraum 11 zwischen Conjunktiva 4 und Sclera 2 (Fig. 1a, links) und die Drainage aus der Vorderkammer 10 des Auges in den in den Suprachoroidalraum 12 zwischen Sclera 2 und Choroidea 5 (Fig. 1b, rechts).

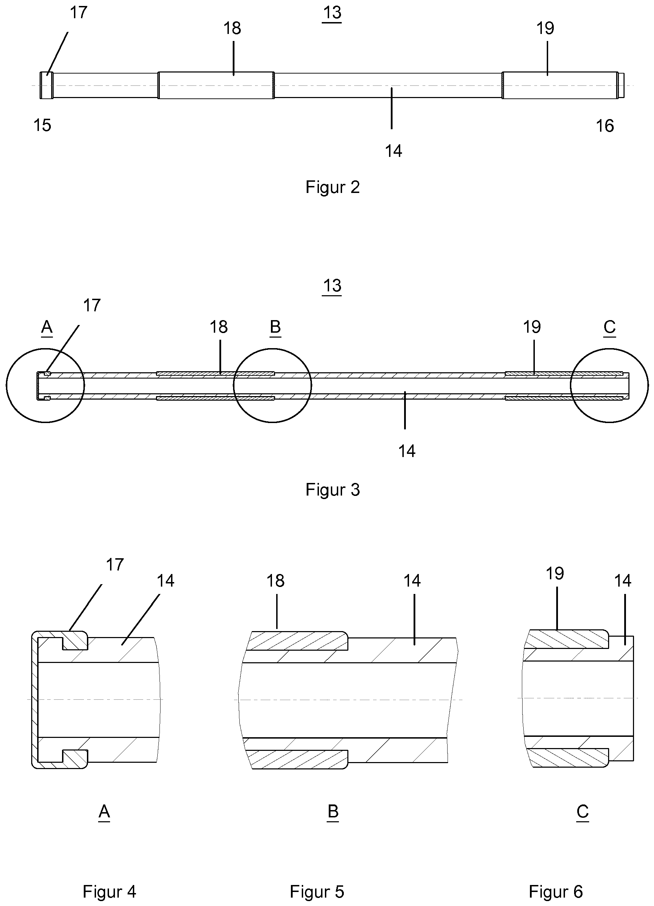

Verschiedene Ausführungsformen des Glaukom-Drainage-Implantats 13 werden in den Figuren 2 bis 10 gezeigt. Es besteht aus einem flexiblen, länglichen, beispielsweise zylindrischen Grundkörper 14, welcher innen hohl ist und in verschiedene Abschnitte eingeteilt ist und ein vorderes Ende 15 (Einlassende) und ein hinteres Ende 16 (Auslassende) aufweist.

Eine erste Ausführungsform des erfindungsgemäßen Glaukom-Drainage-Implantats 13 wird in den Figuren 2 bis 6 dargestellt. Ein erster Abschnitt A befindet sich am vorderen Ende 15. Hier ist zur Vermeidung einer Hypotonie im Einstrombereich des Implantats 13 eine flusslimitierende, vorzugsweise biodegradierbare, semipermeable Membran 17 angeordnet, welche das axiale Einstromlumen bedeckt. In der Detailansicht A in Figur 4 ist dies deutlicher gezeigt. Die Auslegung der flusslimitierenden Membran 17 erfolgt für eine maximale Kammerwasserdrainage von z.B. 2 µl/min bei einer Druckdifferenz von z.B. 15 mmHg (Subconjunctivalraum) bzw. z.B. 2 mmHg (Suprachoroidalraum) zum Zeitpunkt der Implantation. Wochen nach der Implantation hat sich im Ausstrombereich i.d.R. eine fibröse Gewebekapsel um das Implantat 13 gebildet, die dann den Hauptwiderstand der Drainage darstellt. Daher kann mit zunehmender Implantationszeit eine Degradation der flusslimitierenden Membran 17 erfolgen, wodurch der Strömungswiderstand des Implantats 13 sukzessive abnimmt. Durch den Ausgleich des zunehmenden Strömungswiderstands der fibrösen Gewebekapsel durch den abnehmenden Strömungswiderstand des Implantats 13 können eine annähernd konstante Kammerwasserdrainage von z.B. 2 µl/min und somit eine Senkung des intraokularen Drucks gewährleistet werden.

Der zweite Abschnitt B befindet sich auf dem Glaukom-Drainage-Implantat 13 auf ca. einem Drittel des rohrförmigen Grundkörpers 14 vom vorderen Ende 15 aus gesehen. Es handelt sich hier um ein optionales Fixierungselement 18, welches für die optimale Einheilung bzw. Fixierung des Implantats 13 im Gewebe verwendet wird. Das Fixierungselement 18 ist vorzugsweise zirkulär über den gesamten Umfang des Implantats 13 angeordnet. Alternativ ist auch eine Verteilung eines, aus zwei oder mehreren Elementen bestehenden Fixierungselements 18 in einzelnen Abschnitten über den Umfang des Implantats 13 möglich.

Der dritte Abschnitt C ist am hinteren Ende 16 angeordnet und ist ein an das Glaukom-Drainage- Implantat 13 angekoppeltes Local-Drug-Delivery-System 19 (LDD) zur Vermeidung der Bildung einer fibrösen Gewebekapsel. Das Local-Drug-Delivery-System 19 ist vorzugsweise zirkulär über den gesamten Umfang des Implantats 13 angeordnet. Alternativ ist auch eine Verteilung eines, aus zwei oder mehreren Elementen bestehenden Local-Drug-Delivery-Systems 19 in einzelnen Abschnitten über den Umfang des Implantats 13 möglich.

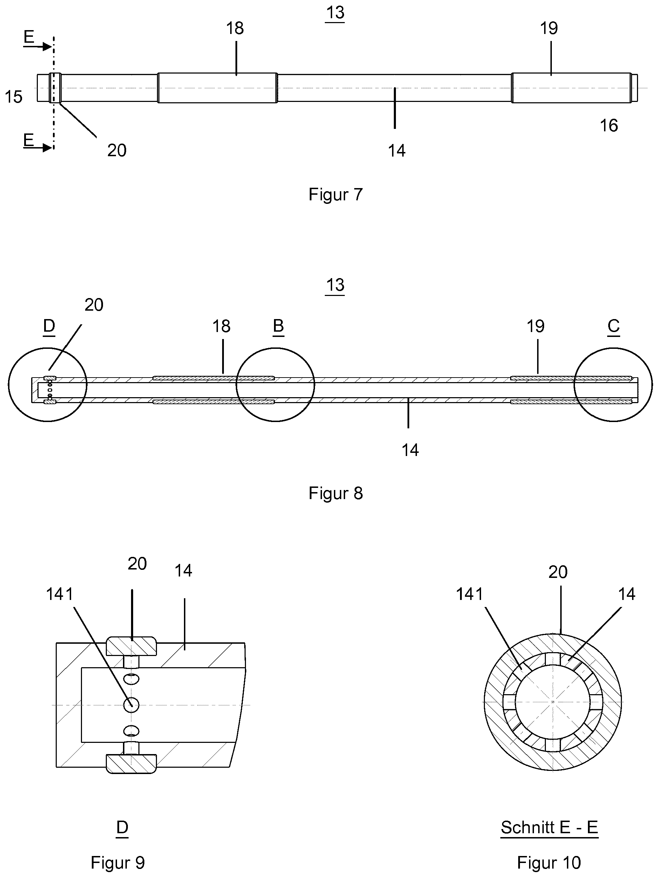

Die Figuren 7 bis 10 stellen die zweite Ausführungsform des erfindungsgemäßen Glaukom-Drainage-Implantats13 dar. Auch hier kommt eine flusslimitierende, vorzugsweise biodegradierbare, semipermeable Membran 20 im Einstrombereich des Implantats 13 zur Anwendung. Die Figur 9 zeigt den Abschnitt D als Detailansicht, welcher im Wesentlichen dem Abschnitt A aus Figur 4 entspricht. Der Grundkörper 14 des Glaukom-Drainage- Implantats 13 ist entgegen zu Figur 4 an seinem vorderen Ende 15 geschlossen. Die Membran 20 ist beabstandet von dem vorderen Ende 15 in den Grundkörper 14 direkt eingearbeitet. In Figur 10 ist ein Schnitt E-E durch das Implantat 13 mit seinem Grundkörper 14 und der Membran 20 zu sehen. Um den Fluss des Kammerwassers limitieren zu können, sind um den Umfang des Grundkörpers 14 verteilt Bohrungen 141 als radiale Einstromöffnungen angeordnet, welche durch die darüber liegende Membran 20 abgedeckt werden. Dadurch kann auch nach der vollständigen Degradation der Membran 20 ein zur Vermeidung einer Hypotonie erforderlicher Strömungswiderstand aufrechterhalten werden.

Alternativ ist sowohl für die erste als auch die zweite Ausführungsform des Glaukom-Drainage-Implantats 13 eine biostabile, also nicht biodegradierbare, Ausführung der flusslimitierenden, semipermeablen Membran denkbar. Diese Ausführungsform sollte aber vorzugsweise in Kombination mit dem an das Glaukom-Drainage-Implantat 13 angekoppelten Local-Drug-Delivery-System 19 (LDD) zur Vermeidung der Bildung einer fibrösen Gewebekapsel zur Anwendung kommen.

Als Verfahren zur Herstellung für die flusslimitierenden Membranen 17 und 20 kommt vorzugsweise das Electrospinning zur Anwendung. Dieses Verfahren ermöglicht ein direktes Bespinnen des Implantat-Grundkörpers 14 und eine exakte Einstellung des Strömungswiderstands durch die Variation verschiedener Prozessparameter. Mit diesem Verfahren können sowohl die, das axiale Einstromlumen bedeckende Membran 17, als auch die, die zusätzlich geschaffenen radialen Einstromöffnungen bedeckende Membran 20 hergestellt werden. Als Material für die flusslimitierenden, biodegradierbaren Membranen 17 und 20 wird beispielsweise der Polymerwerkstoff Poly(4-hydroxybuttersäure) verwendet.

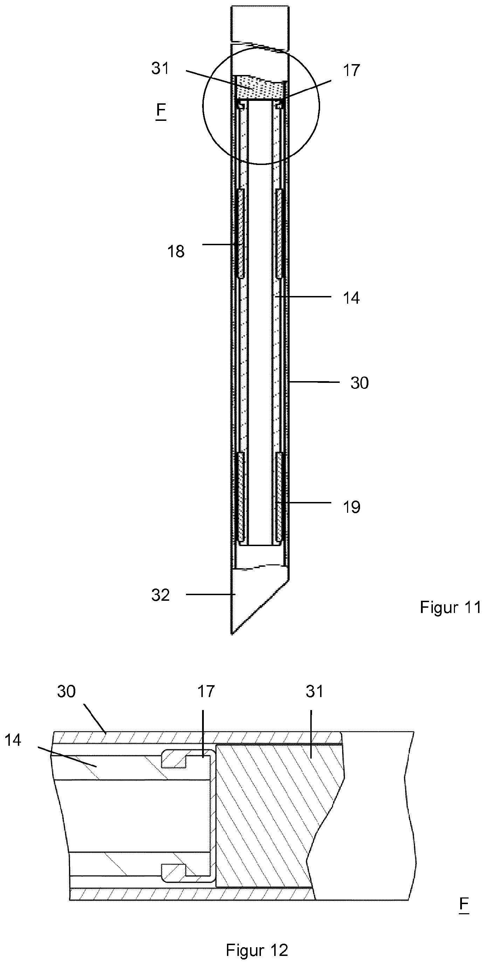

Das Electrospinning-Verfahren ist weiterhin potenziell zur Herstellung weiterer Komponenten des Implantats, wie dem Local-Drug-Delivery-System 19 oder dem Fixierungselement 18 geeignet. Hierbei können beliebige Polymermaterialien auch in Verbindung mit entsprechenden Medikamenten versponnen werden. Zur Fibrosehemmung ist für das Local-Drug-Delivery-System 19 z.B. der aus der Trabekulektomie bekannte Wirkstoff Mitomycin C interessant. Die lokale Förderung der Zelladhäsion und die daraus resultierende optimale Einheilung im Gewebe könnte für das Fixierungselement 18 z.B. über Integrin-RGD Interaktion erfolgen. Der Implantat-Grundkörper 14 besteht vorzugsweise aus einem Elastomer, z.B. Silikon oder Polyurethan. Die Form des Implantats 13 ist vorzugsweise zylindrisch, so dass eine minimalinvasive Implantation mit Hilfe eines Injektors 30 ermöglicht wird. Der Injektor 30 mit einem darin enthaltenem Glaukom-Drainage-Implantat 13 wird in den Figuren 11 und 12 dargestellt. Für dieses Ausführungsbeispiel wird das Glaukom-Drainage- Implantat 13 mit dem flexiblen, vorzugsweise zylindrischen Grundkörper 14, der flusslimitierenden, vorzugsweise biodegradierbaren, semipermeablen Membran 17, dem Fixierungselement 18 und dem Local-Drug-Delivery-System 19 für die Implantation vorbereitet. Der flexible Injektor 30 ist in dieser Ausführungsform an seinem distalen Ende beispielsweise mit einer scharfen Spitze 32 ausgeführt, um ein Durchdringen der okulären Gewebe zu erleichtern. An seinem proximalen Ende ist ein Stempel 31 in den Injektor 30 eingebracht, mit dessen Hilfe das Vorschieben des Glaukom-Drainage- Implantats 13 an den gewünschten Implantationsort erfolgt. Das Glaukom-Drainage-Implantat 13 wird in den Injektor 30 geladen, wobei die Membran 17 oder 20 der Spitze 32 des Injektors 30 gegenüberliegt. Der Injektor 30 wird mit einem Stempel 31 verschlossen, welcher gleichzeitig für den Vortrieb des Glaukom-Drainage-Implantats 13 in das Augeninnere genutzt wird. Figur 12 zeigt eine Detailansicht F aus Figur 11, in welcher die Lage des Glaukom-Drainage-Implantats 13 vor dem Stempel 31 verdeutlicht wird.

Um eine glatte Außenoberfläche des Grundkörpers 14 zu erreichen und damit eine minimalinvasive Implantation zu begünstigen, sind die Funktionselemente flusslimitierende Membran 17 und 20, Local-Drug-Delivery-System 19 und Fixierungselement 18 in entsprechende lokale Nuten in die Wandfläche des Grundkörpers 14 integriert.

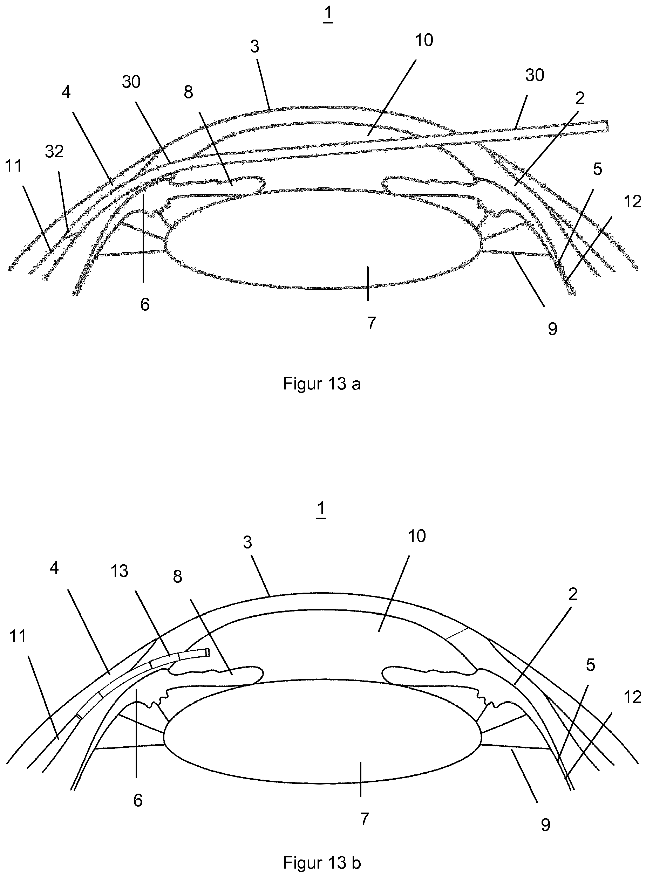

Ein mögliches minimalinvasives Implantationsverfahren ist in Figur 13 veranschaulicht, wobei die einzelnen Zeichnungen a und b die verschiedenen Phasen der Implantation schematisieren. Fig. 13a zeigt das Auge 1 während des Eingriffs, also den Zeitpunkt der Implantation. Dazu wird der Injektor 30 mit dem Glaukom-Drainage-Implantat 13 über eine kleine Inzision der Cornea 3 bis zum Implantationsort, hier dem Subconjunctivalraum, vorgeschoben. Mit Hilfe eines Stempels 31 wird das Implantat 13 aus dem Injektor 30 freigesetzt. Figur 13b zeigt die schematische Anordnung des freigesetzten Glaukom-Drainage- Implantats 13 am Implantationsort.

### Bezugszeichen

- 1: Auge
- 2: Sclera
- 3: Hornhaut (Cornea)
- 4: Bindehaut (Conjunktiva)
- 5: Aderhaut (Choroidea)
- 6: Ziliarkörper
- 7: Linse
- 8: Iris
- 9: Zonulafasern
- 10: Vorderkammer
- 11: Subconjunctivalraum
- 12: Suprachoroidalraum
- 13: Glaukom-Drainage-Implantat
- 14: länglicher hohler Grundkörper
141 Bohrungen
- 15: vorderes Ende (Einlassende)
- 16: hinteres Ende (Auslassende)
- 17: Membran
- 18: Fixierungselement
- 19: Local-Drug-Delivery-System
- 20: Membran

- 30: Injektor
- 31: Stempel
- 32: Spitze

## Patentansprüche

1. Glaukom-Drainage-Implantat, welches aus einem länglichen, hohlen Grundkörper (14) besteht, bei welchem im Einstrombereich des Implantats (13) eine flusslimitierende Membran (20) angeordnet ist, wobei an einem vorderen, geschlossenen Ende (15) des Grundkörpers (14) um seinen Umfang verteilt Bohrungen (141) angeordnet sind, welche durch die darüber liegende Membran (20) abgedeckt werden,
**dadurch gekennzeichnet, dass** lokale Nuten in der Wandfläche des Grundkörpers (14) zur Aufnahme der Membran (20), eines Fixierungselements (18) und eines Local-Drug-Delivery-Systems (19) entsprechend eingelassen sind.

2. Glaukom-Drainage-Implantat nach Anspruch 1 **dadurch gekennzeichnet, dass** auf ca. einem Drittel des länglichen, hohlen Grundkörpers (14) von einem vorderen Ende (15) aus gesehen das Fixierungselement (18) angeordnet ist, welches zur Fixierung des Implantats (13) im Gewebe dient.

3. Glaukom-Drainage-Implantat nach Anspruch 1 oder 2 **dadurch gekennzeichnet, dass**
zur Herstellung des Fixierungselements (18) ein Fertigungsverfahren in Form von Electrospinning zur Anwendung kommt.

4. Glaukom-Drainage-Implantat nach Anspruch 1 **dadurch gekennzeichnet, dass** an einem hinteren Ende (16) des länglichen, hohlen Grundkörpers (14) das Local-Drug-Delivery-System (19) angeordnet ist, welches zur Vermeidung einer Bildung einer fibrösen Gewebekapsel dient.

5. Glaukom-Drainage-Implantat nach Anspruch 1 oder 4 **dadurch**
**gekennzeichnet, dass**
zur Herstellung des Local-Drug-Delivery-Systems (19) ein Fertigungsverfahren in Form von Electrospinning zur Anwendung kommt.

6. Glaukom-Drainage-Implantat nach Anspruch 1 **dadurch gekennzeichnet, dass** die Membran (20) aus biodegradierbarem, semipermeablem Material besteht.

7. Glaukom-Drainage-Implantat nach Anspruch 1 **dadurch gekennzeichnet, dass** die Membran (20) aus einem Polymerwerkstoff besteht.

8. Glaukom-Drainage-Implantat nach Anspruch 1 **dadurch gekennzeichnet, dass** die Membran (20) aus Poly(4-hydroxybuttersäure) besteht.

9. Glaukom-Drainage-Implantat nach Anspruch 1 **dadurch gekennzeichnet, dass** die Membran (20) mit zunehmender Implantationszeit einen abnehmenden Strömungswiderstand aufweist.

10. Glaukom-Drainage-Implantat nach Anspruch 1 **dadurch gekennzeichnet, dass** die Membran (20) aus biostabilem, semipermeablem Material besteht.

11. Glaukom-Drainage-Implantat nach einem der Ansprüche 1 oder 6 bis 10
**dadurch gekennzeichnet, dass**
zur Herstellung der Membran (20) ein Fertigungsverfahren in Form von Electrospinning zur Anwendung kommt.

## Claims

1. Glaucoma drainage implant composed of an elongate, hollow base body (14) in which a flow-limiting membrane (20) is arranged in the inflow region of the implant (13), and in which holes (141) are distributed about the circumference of the base body (14) at a front, closed end (15) thereof, said holes (141) being covered by the membrane (20) lying over them, **characterized in that** local grooves are correspondingly recessed in the wall surface of the base body (14) in order to receive the membrane (20), a fixing element (18) and a local drug delivery system (19).

2. Glaucoma drainage implant according to Claim 1, **characterized in that** the fixing element (18), which serves to fix the implant (13) in the tissue, is arranged on approximately one third of the elongate, hollow base body (14), as seen from a front end (15).

3. Glaucoma drainage implant according to Claim 1 or 2, **characterized in that** a production method in the form of electrospinning is used to produce the fixing element (18).

4. Glaucoma drainage implant according to Claim 1, **characterized in that** the local drug delivery system (19), which serves to avoid formation of fibrous tissue capsule, is arranged at a rear end (16) of the elongate, hollow base body (14).

5. Glaucoma drainage implant according to Claim 1 or 4, **characterized in that** a production method in the form of electrospinning is used to produce the local drug delivery system (19).

6. Glaucoma drainage implant according to Claim 1, **characterized in that** the membrane (20) is made from biodegradable, semi-permeable material.

7. Glaucoma drainage implant according to Claim 1, **characterized in that** the membrane (20) is made from a polymer material.

8. Glaucoma drainage implant according to Claim 1, **characterized in that** the membrane (20) is made from poly(4-hydroxybutyric acid).

9. Glaucoma drainage implant according to Claim 1, **characterized in that** the membrane (20) has a flow resistance that decreases as the implantation time increases.

10. Glaucoma drainage implant according to Claim 1, **characterized in that** the membrane (20) is made from biostable, semi-permeable material.

11. Glaucoma drainage implant according to one ofClaims 1 or 6 to 10, **characterized in that** a production method in the form of electrospinning is used to produce the membrane (20).

## Revendications

1. Implant de drainage de glaucome, constitué d'un corps de base allongé (14), dans lequel est disposée une membrane limitant l'écoulement (20) dans la région d'afflux de l'implant (13), des orifices (141) étant disposés au niveau d'une extrémité avant fermée (15) du corps de base (14) de manière répartie autour de sa périphérie, lesquels sont recouverts par la membrane (20) placée sur ceux-ci,
**caractérisé en ce que** des rainures locales sont réalisées de manière correspondante dans la surface de paroi du corps de base (14) pour recevoir la membrane (20), un élément de fixation (18) et un système de distribution topique de médicament (19) .

2. Implant de drainage de glaucome selon la revendication 1, **caractérisé en ce que** l'élément de fixation (18) est disposé approximativement à un tiers du corps de base allongé (14), vu depuis une extrémité avant (15), lequel élément de fixation sert à la fixation de l'implant (13) dans le tissu.

3. Implant de drainage de glaucome selon la revendication 1 ou 2, **caractérisé en ce que** pour la fabrication de l'élément de fixation (18), on utilise un procédé de fabrication par électrospinning.

4. Implant de drainage de glaucome selon la revendication 1, **caractérisé en ce qu'**à une extrémité arrière (16) du corps creux allongé (14) est disposé le système de distribution de médicament topique (19) qui sert à éviter une formation d'une capsule de tissu fibreuse.

5. Implant de drainage de glaucome selon la revendication 1 ou 4, **caractérisé en ce que** pour la fabrication du système de distribution de médicament topique (19), on utilise un procédé de fabrication par électrospinning.

6. Implant de drainage de glaucome selon la revendication 1, **caractérisé en ce que** la membrane (20) se compose d'un matériau biodégradable semi-perméable.

7. Implant de drainage de glaucome selon la revendication 1, **caractérisé en ce que** la membrane (20) se compose d'un matériau polymère.

8. Implant de drainage de glaucome selon la revendication 1, **caractérisé en ce que** la membrane (20) se compose d'acide poly(4-hydroxybutyrique).

9. Implant de drainage de glaucome selon la revendication 1, **caractérisé en ce que** la membrane (20) présente une résistance à l'écoulement décroissante avec l'augmentation de la durée d'implantation.

10. Implant de drainage de glaucome selon la revendication 1, **caractérisé en ce que** la membrane (20) se compose d'un matériau biostable, semi-perméable.

11. Implant de drainage de glaucome selon l'une quelconque des revendications 1 ou 6 à 10, **caractérisé en ce que** pour la fabrication de la membrane (20), on utilise un procédé de fabrication par électrospinning.
